# FASCICULE DE BREVET EUROPEEN

(11) **EP 0 561 710 B1**
(45) Date de publication et mention de la délivrance du brevet: **08.01.1997**
(21) Numéro de dépôt: 93400710.5
(22) Date de dépôt: 19.03.1993
(51) Int. Cl.: A61F 2/08

(54) **Ligament artificiel**
Kunstband
Artificial ligament

(30) Priorité: 19.03.1992 FR 9203307
(43) Date de publication de la demande: 22.09.1993
(73) Titulaire: Laboureau, Jacques-Philippe, F-21000 Dijon (FR)
(72) Inventeur: Laboureau, Jacques-Philippe, F-21000 Dijon (FR)
(74) Mandataire: Bruder, Michel

(56) Documents cités:
- EP-A- 0 255 408
- EP-A- 0 437 174
- WO-A-88/06026
- FR-A- 2 651 994
- US-A- 4 917 700
- US-A- 5 004 474
- US-A- 5 024 669

## Description

La présente invention concerne un ligament artificiel pour le remplacement de ligaments naturels articulaires.

On connaît, par exemple par le brevet EP-255 408, un ligament artificiel pour le remplacement de ligaments articulaires. L'originalité de ce ligament antérieur, qui est enrobé d'une résine visco-élastique biocompatible sur la partie de l'âme destinée à être implantée dans la partie intra-articulaire, réside essentiellement en ce que, avant imprégnation par ladite résine, les fibres synthétiques longitudinales adjacentes constituant ledit ligament sont maintenues solidaires les unes des autres par une colonne de mailles transversales, du type à mailles jetées. Ce ligament est donc généralement obtenu par roulage sur elle-même d'une bande de tricot fabriquée sur un métier de type Rachel.

Bien que les qualités dynanométriques du ligament précédent soient reconnues, on a mis en évidence que le tricotage de la zone intra-articulaire pouvait s'avérer néfaste à la résistance à la traction. De même, alors que la rugosité du ligament tricoté favorise, à ses extrémités implantées dans l'os, la repousse osseuse, l'enrobage de l'âme par la résine empêche la réhabitation par les fibroblastes. De ce fait, le ligament proposé dans le brevet sus-mentionné est une prothèse pure.

La présente invention vise à remédier à ces inconvénients en proposant un nouveau ligament artificiel pour le remplacement d'un ligament naturel articulaire, formé par roulage ou par pliage sur elle-même d'une laize comprenant deux parties intra-osseuses venant de part et d'autre d'au moins une partie intra-articulaire médiane, caractérisé en ce que ladite partie intra-articulaire est formée de fibres techniques adjacentes, non liées entre elles, et en ce que lesdites parties intra-osseuses sont formées par un tricotage-chaîne des fibres techniques à l'aide de fils de trame dont le serrage évite tout déplacement relatif desdites fibres techniques par rapport aux mailles du tricot.

Le ligament ainsi obtenu forme donc, dans sa partie intra-articulaire, un écheveau de fibres parfaitement libres les unes par rapport aux autres et dont l'espace intersticiel peut être totalement recolonisé par le tissu conjonctif vivant du sujet opéré ; par conséquent, le ligament conforme à l'invention remplit un rôle de tuteur fibreux beaucoup plus avantageux que celui d'une prothèse pure, du type de celle décrite dans le brevet EP-255 408 déjà mentionné.

Par ailleurs, on a pu vérifier que, contrairement à l'opinion jusqu'alors admise par l'homme du métier ordinaire, cet écheveau de fibres libres s'avérait plus résistant à la rupture en traction qu'un faisceau de fibres tissées ou tricotées. Il est à signaler que cette augmentation de la résistance à la rupture n'est pas négligeable. On doit en effet comprendre qu'un ligament artificiel n'est pas sensible, contrairement à un ligament naturel, à l'effet proprioceptif qui se traduit par le fait que, lorsqu'une articulation est sollicitée, les muscles qui s'y attachent contribuent à limiter la tension exercée sur les ligaments articulaires en réponse à leur sollicitation ; en conséquence, il est souhaitable qu'un ligament artificiel soit beaucoup plus résistant que le ligament naturel qu'il remplace.

Le ligament artificiel conforme à l'invention présente aussi l'avantage que, du fait de sa structure intra-articulaire non tissée et non tricotée, il est possible d'augmenter sensiblement le nombre de fibres actives tout en conservant le même encombrement qu'un ligament tricoté ou tissé ; on peut ainsi pratiquement doubler le nombre de fibres actives du ligament.

En outre, cette disposition plus compacte des fibres actives permet de rapprocher ces dernières de la "fibre neutre" du ligament ; on rappelle que cette fibre neutre est, par définition, la fibre qui est située sur l'axe isométrique passant par les points isométriques des deux os de l'articulation concernée, par exemple le genou. L'intérêt de cette fibre neutre est qu'il s'agit d'une fibre dont la longueur ne varie pas, ou peu, entre deux positions distinctes de l'articulation concernée, c'est-à-dire lors d'un mouvement de flexion ou d'extension ; par conséquent, un grand nombre de fibres du ligament artificiel proposé, en étant proches de la fibre neutre, varient peu en longueur. De ce fait, on peut s'approcher des conditions d'isométrie idéale recherchées lors des opérations de plastie articulaire, notamment du genou.

D'un autre côté, le tricotage des parties intra-osseuses du ligament artificiel conforme à l'invention permet d'une part, par un choix judicieux du noeud et de la maille, de rendre rugueuses lesdites parties intra-osseuses du ligament, avec la conséquence de favoriser la fixation osseuse et, d'autre part, de donner une configuration hyperstatique au faisceau des fibres médianes non tricotées. A cet égard, le type de maille et la capacité de serrage des extrémités du ligament par les fils de trame jouent un rôle fondamental. En particulier, il convient de préciser que, selon l'invention, il ne suffirait pas de tendre un écheveau de fibres entre deux dispositifs de fixation osseuse quelconques pour obtenir tous les avantages précités ; en effet, un ligament exclusivement multifilamentaire verrait chacune de ses fibres constitutives supporter individuellement toute la tension appliquée, sans qu'un effet de maintien collectif soit possible. Suivant la présente invention, les déplacements relatifs d'une fibre technique du ligament tendent à resserrer les noeuds formés autour des fibres adjacentes par les fils de trame, d'où un effet de compensation globale des tensions subies par le ligament. Cet effet s'avère particulièrement efficace lorsque la distribution des efforts est anisotrope, cas pour lequel un faisceau simplement multifilamentaire verraient ses fibres les plus sollicitées se rompre (fibres les plus éloignées de la fibre neutre).

D'autres caractéristiques et avantages de la présente invention ressortiront mieux de la description qui va suivre d'un mode d'exécution d'un ligament articulaire donné à titre d'exemple non limitatif en référence aux dessins annexés sur lesquels :
- la figure 1 est une vue en perspective en plan de la laize servant, par roulage, à former le ligament,
- la figure 2 est une vue en perspective du ligament roulé et cousu sur sa dernière spire concentrique,
- la figure 3 est une vue d'un ligament de remplacement du ligament croisé antérieur implanté dans l'articulation du genou.

Le ligament conforme à l'invention est fabriqué à partir d'une laize 1 formée de deux bandes latérales tricotées 2 venant de part et d'autre d'une bande centrale 3, non tricotée, où n'apparaissent donc que des fibres techniques longitudinales. Préférentiellement, ce tricot composite alterné est obtenu sur un métier dont les fils techniques constituent les fils transversaux (rangées), les colonnes médianes du tricot n'étant donc pas tricotées. Les fils de trame et les fils techniques transversaux employés pour ce tricot sont des fibres de polyester dèjà employés pour la fabrication d'autres ligaments prothétiques et, par exemple, il s'agira de fils texturés fins, titrés entre 100 et 200 décitex.

Le cas échéant, certains fils pourront être imprégnés d'un agent mouillant radio-opaque, de manière à faciliter la détection par radiographie du ligament implanté dans l'articulation.

Selon un mode de réalisation de l'invention, le tricotage des bandes latérales 2 du ligament est tel qu'une fibre technique subit, dans les parties intra-osseuses, un déport latéral n'excédant pas le quart de son diamètre, et généralement pas plus du sixième. Chaque fibre est donc sollicitée sur toute sa longueur, y compris les parties enchassées dans les parties intra-osseuses.

En outre, on a choisi une maille telle qu'au moins 10 fibres techniques puissent être logées dans une largeur de la laize 1 égale à 1 centimètre.

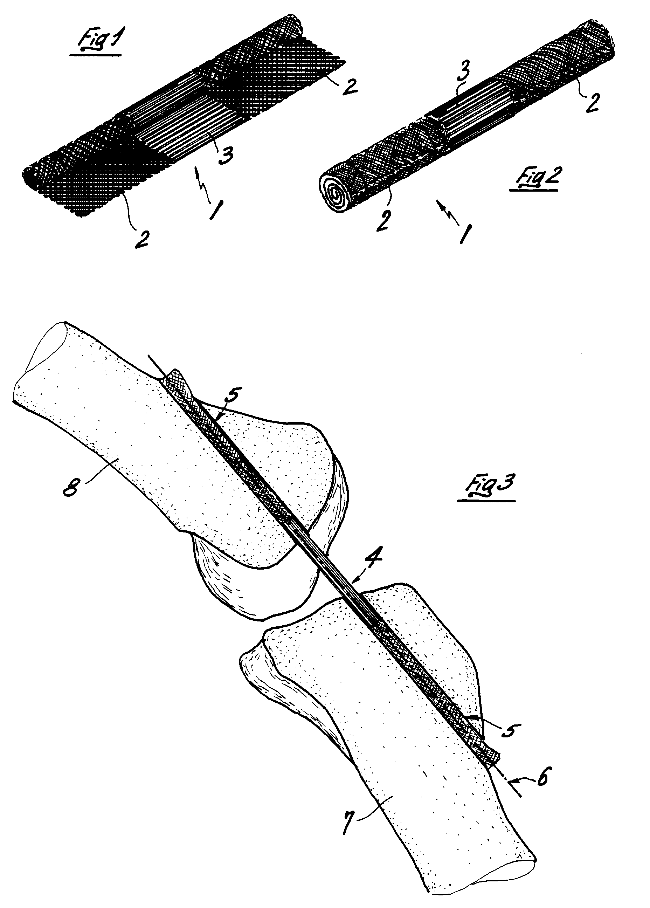

Suivant la figure 2, la laize 1 est roulée sur-elle parallèlement aux fils techniques, puis cousue suivant la lisière de sa dernière spire ; cette technique de roulage convolute à un seul enroulement n'est pas obligatoire et il pourra être envisagé d'enrouler la laize 1 à partir de ses deux lisières simultanément. La partie intra-articulaire 4 se présente alors comme un écheveau de fibres techniques non liées entre elles, susceptibles de glisser librement les unes par rapport aux autres avec une amplitude limitée par le "rappel" dû aux fils de trame, qui sont noués autour desdites fibres techniques dans les parties intra-osseuses 5 aux extrémités du ligament.

Suivant la longueur de la laize 1 que l'on a enroulée, on peut obtenir tout type de ligament pour le remplacement des ligaments articulaires les plus courants, tels que les ligaments croisés postéro-interne et antéro-externe du genou. Le diamètre des ligaments artificiels obtenus varie alors entre 4,5 et 7 millimètres, avec un nombre de fibres techniques dépendant proportionnellement de la largeur de la laize 1 enroulée.

A cet égard, il est important de remarquer, conformément à la figure 3, que les fibres techniques longitudinales doivent former un faisceau dense et compacte dans la partie intra-articulaire médiane 4 du ligament, en étant situées le plus près possible d'une fibre dite "neutre" qui est implantée suivant l'axe isométrique 6 passant par les points isométriques des os de l'articulation concernée, par exemple le tibia 7 et le fémur 8 dans le cas du genou.

Subsidiairement, on peut imprégner la partie intra-articulaire 4 du ligament d'un ou de plusieurs agents biochimiques favorisant la colonisation par les fibroblastes, c'est-à-dire la reconstruction d'un ligament naturel de même extension que le ligament artificiel implanté qui sert alors de tuteur biologique à cette reconstruction.

Enfin, bien que, du fait de la rugosité d'une face du tricot des bandes latérales 2 de la laize 1, la liaison mécanique du ligament avec la paroi des tunnels osseux d'implantation soit excellente, il est proposé d'enduire ou d'imprégner les parties intra-osseuses dudit ligament d'un ou de plusieurs facteurs de repousse osseuse tel que l'hydroxyapatite.

## Revendications

1. Ligament artificiel pour le remplacement d'un ligament naturel articulaire, formé par roulage ou par pliage sur elle-même d'une laize (1) comprenant deux parties intra-osseuses (2, 5) venant de part et d'autre d'au moins une partie intra-articulaire médiane (3, 4), caractérisé en ce que ladite partie intra-articulaire (3, 4) est formée de fibres techniques adjacentes, non liées entre elles, et en ce que lesdites parties intra-osseuses (2, 5) sont formées par un tricotage-chaîne des fibres techniques à l'aide de fils de trame dont le serrage évite tout déplacement relatif desdites fibres techniques par rapport aux mailles du tricot.

2. Ligament artificiel selon la revendication précédente, caractérisé en ce que le tricotage des extrémités du ligament est tel qu'une fibre technique subit, dans les parties intra-osseuses (2, 5), un déport latéral n'excédant pas le quart de son diamètre.

3. Ligament artificiel selon la revendication précédente, caractérisé en ce que le tricotage des extrémités du ligament est tel qu'une fibre technique subit, dans les parties intra-osseuses (2, 5), un déport latéral n'excédant pas le sixième de son diamètre.

4. Ligament artificiel selon l'une quelconque des revendications précédentes, caractérisé en ce que la laize (1) formant par roulage ou par pliage ledit ligament comporte au moins 10 fibres techniques par centimètre.

5. Ligament artificiel selon l'une quelconque des revendications précédentes, caractérisé en ce que les parties intra-osseuses (2, 5) sont enrobées, ou simplement imprégnées, d'un ou de plusieurs facteurs de repousse osseuse.

6. Ligament artificiel selon l'une quelconque des revendications précédentes, caractérisé en ce que les fibres techniques de la partie intra-articulaire médiane (3, 4) sont imprégnées d'un ou de plusieurs agents biochimiques favorisant la colonisation par les fibroblastes.

7. Ligament artificiel selon l'une quelconque des revendications précédentes, caractérisé en ce que certaines des fibres techniques de la partie intra-articulaire médiane (3, 4) sont radio-opaques.

8. Ligament artificiel selon l'une quelconque des revendications précédentes, caractérisé en ce que les fibres techniques longitudinales forment un faisceau dense et compact dans la partie intra-articulaire médiane (3, 4), en étant situées le plus près possible d'une fibre dite neutre qui est à implanter suivant l'axe isométrique (6) passant par les points isométriques des os de l'articulation concernée.

## Claims

1. Artificial ligament for the replacement of a natural ligament in a joint, formed by rolling up or folding over itself a strip (1) of material comprising two intra-osseous portions (2, 5) located on either side of at least one median intra-articular section (3, 4), characterized in that the said intra-articular section (3, 4) is formed of adjacent technical fibres which are not interconnected, and in that the said intra-osseous portions (2, 5) are formed by a chain-knit of the technical fibres with weft threads whose tightness prevents any relative movement of the said technical fibres with respect to the mesh of the knitted fabric.

2. Artificial ligament according to the preceding Claim, characterized in that the knitting of the ends of the ligament is such that in the intra-osseous portions (2, 5), a technical fibre undergoes lateral deflection by no more than one-quarter of its diameter.

3. Artificial ligament according to the preceding Claim, characterized in that the knitting of the ends of the ligament is such that in the intra-osseous portions (2, 5), a technical fibre undergoes lateral deflection by no more than one-sixth of its diameter.

4. Artificial ligament according to any of the preceding Claims, characterized in that the strip (1) of material rolled up or folded over to form the said ligament comprises at least 10 technical fibres per centimetre.

5. Artificial ligament according to any of the preceding Claims, characterized in that the intra-osseous portions (2, 5) are coated or simply impregnated with one or more agents which promote bone growth.

6. Artificial ligament according to any of the preceding Claims, characterized in that the technical fibres in the median intra-articular section (3, 4) are impregnated with one or more biochemical agents which promote colonization by fibroblasts.

7. Artificial ligament according to any of the preceding Claims, characterized in that some of the technical fibres in the median intra-articular section (3, 4) are radio-opaque.

8. Artificial ligament according to any of the preceding Claims, characterized in that the longitudinal technical fibres form a dense and compact bundle in the median intra-articular section (3, 4), and are situated as close as possible to a fibre, designated the neutral fibre, which should be implanted along the isometric axis (6) passing through the isometric points of the bones of the joint concerned.

## Patentansprüche

1. Kunstband als Ersatz für ein natürliches Gelenkband, hergestellt durch Aufrollen oder Falten einer Bahn (1), mit mindestens einem mittleren, im Gelenk liegenden Bereich (3, 4) und zwei beiderseits davon vorgesehenen intraossalen Bereichen (2, 5), dadurch gekennzeichnet, daß der Gelenkbereich (3, 4) aus aneinander angrenzenden, miteinander nicht verbundenen technischen Fasern besteht und die intraossalen Bereiche (2, 5) aus einem Kettengewirk aus technischen Fasern mit Schußfäden bestehen, deren dichte Anordnung jegliche Verschiebung der technischen Fasern gegenüber den Maschen des Gewirks verhindert.

2. Kunstband nach dem vorhergehenden Anspruch, dadurch gekennzeichnet, daß das die Enden des Bandes bildende Gewirk so ausgebildet ist, daß eine technische Faser in den intraossalen Bereichen (2, 5) sich um höchstens ein Viertel ihres Durchmessers seitlich verschieben kann.

3. Kunstband nach dem vorhergehenden Anspruch, dadurch gekennzeichnet, daß das die Enden des Bandes bildende Gewirk so ausgebildet ist, daß eine technische Faser in den intraossalen Bereichen (2, 5) sich um höchstens ein Sechstel ihres Durchmessers seitlich verschieben kann.

4. Kunstband nach einem der vorhergehenden Ansprüche, dadurch gekennzeichnet, daß die Bahn (1), aus der das Band durch Falten oder Rollen hergestellt ist, mindestens 10 technische Fasern pro Zentimeter aufweist.

5. Kunstband nach einem der vorhergehenden Ansprüche, dadurch gekennzeichnet, daß die intraossalen Bereiche (2, 5) mit einem oder mehreren knochenwachstumsfördernden Faktoren umhüllt oder einfach getränkt sind.

6. Kunstband nach einem der vorhergehenden Ansprüche, dadurch gekennzeichnet, daß die technischen Fasern des mittleren Gelenkbereichs (3, 4) mit einem oder mehreren die Ansiedlung von Fibroblasten begünstigenden biochemischen Stoffen getränkt sind.

7. Kunstband nach einem der vorhergehenden Ansprüche, dadurch gekennzeichnet, daß bestimmte der technischen Fasern des mittleren Gelenkbereichs (3, 4) für Röntgenstrahlen undurchlässig sind.

8. Kunstband nach einem der vorhergehenden Ansprüche, dadurch gekennzeichnet, daß die längsgerichteten technischen Fasern im mittleren Gelenkbereich (3, 4) ein dichtes und kompaktes Bündel bilden und dabei möglichst dicht neben einer sogenannten neutralen Faser liegen, die entlang der durch die isometrischen Punkte der betroffenen Gelenkknochen hindurchgehenden isometrischen Achse (6) zu liegen kommen soll.
